(19) Europäisches Patentamt
European Patent Office
Office européen des brevets

(11) Publication number: **0 454 436 B1**

(12) **EUROPEAN PATENT SPECIFICATION**

(45) Date of publication of patent specification: **13.09.95**

(51) Int. Cl.[6]: **C07D 495/04**, C07D 333/38, A61K 31/55, //(C07D495/04, 333:00,243:00)

(21) Application number: **91303679.4**

(22) Date of filing: **24.04.91**

The file contains technical information submitted after the application was filed and not included in this specification

# (54) Pharmaceutical compounds.

(30) Priority: **25.04.90 GB 9009229**

(43) Date of publication of application:
**30.10.91 Bulletin 91/44**

(45) Publication of the grant of the patent:
**13.09.95 Bulletin 95/37**

(84) Designated Contracting States:
**AT BE CH DE DK ES FR GB GR IT LI LU NL SE**

(56) References cited:
**GB-A- 1 533 235**
**GB-A- 1 533 236**
**GB-A- 1 577 743**
**US-A- 3 953 430**

**CHEMICAL ABSTRACTS, vol. 93, no. 17, 27 October 1980 Columbus, Ohio, USA J.K. CHAKRABARTI et al.: "Heteroarenobenzodiazepines. 3. 4-Piperazinyl-10 H-thieno[2,3-b] [1,5]benzodiazepines as potential neuroleptics" page 21; ref. no. 160947N & J. Med. Chem. 1980, 23[8], 878-84**

(73) Proprietor: **LILLY INDUSTRIES LIMITED**
**Kingsclere Road**
**Basingstoke**
**Hants RG21 2XA (GB)**

(72) Inventor: **Chakrabarti, Jiban Kumar**
**3 Holly Hedge Road,**
**Frimley**
**Camberley,**
**Surrey (GB)**
Inventor: **Hotten, Terrence Michael**
**134 West Heath Road,**
**Cove**
**Farnborough,**
**Hants (GB)**
Inventor: **Tupper, David Edward**
**8 Mildenhall Close,**
**Lower Earley**
**Reading,**
**Berks (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

JOURNAL OF THE CHEMICAL SOCIETY, PERKIN TRANSACTIONS I, 1978, pages 937 - 941; J.K. CHAKRABARTI et al.: "Heteroarene-fused Benzodiazepines. Part 1. Synthesis of Thieno-[2,3-b][1,5]-, -[3,2-b][1,5]-, and -[3,4-b][1,5]-benzodiazepinones"

(74) Representative: **Hudson, Christopher Mark et al**
**Lilly Industries Limited**
**European Patent Operations**
**Erl Wood Manor**
**Windlesham**
**Surrey GU20 6PH (GB)**

## Description

This invention relates to novel organic compounds and the use thereof as pharmaceuticals.

Currently there are many drugs available for the treatment of disorders of the central nervous system. Amongst these drugs is a category known as antipsychotics for treating serious mental conditions such as schizophrenia and schizophreniform illnesses. The drugs available for such conditions are often associated with undesirable side effects, and there is a need for better products that control or eliminate the symptoms in a safer and more effective way. Furthermore, many patients do not respond or only partially respond to present drug treatment, and estimates of such partial- or non-responders vary between 40% and 80% of those treated.

Ever since antipsychotics were introduced it has been observed that patients are liable to suffer from drug-induced extra pyramidal symptoms which include drug-induced Parkinsonism, acute dystonic reactions, akathisia, tardive dyskinesia and tardive dystonia. The Simpson Angus Scale, Barnes Akathisia Rating Scale and Abnormal Involuntary Movement Scale (AIMS) are well known scales for assessing extra pyramidal symptoms. The great majority of drugs available for treatment of schizophrenia are prone to produce these extra pyramidal side effects when used at dosages that yield a beneficial effect on the symptoms of the disease. The severity of adverse events and/or lack of efficacy in a considerable number of patients frequently results in poor compliance or termination of treatment.

Many of the drugs are associated with a sedative effect and may also have an undesirable influence on the affective symptoms of the disease, causing depression. In some instances long term use of the drug leads to irreversible conditions, such as the tardive dyskinesia and tardive dystonia referred to above.

A widely-used antipsychotic, haloperidol, is one such drug, which has been reported as causing a high incidence of extra pyramidal symptoms and may also cause tardive dyskinesia. More recently, clozapine, one of a large group of tricyclic antipsychotics, has been introduced with the claim that it is free from extra pyramidal effects. However, the compound was found to cause agranulocytosis in some patients, a condition resulting in a lowered white blood cell count which can be life-threatening, and it may now only be employed under very strict medical observation and supervision.

A further group of antipsychotic compounds is that described in British Patent 1 533 235. These include thieno-benzodiazepines having the following structural nucleus.

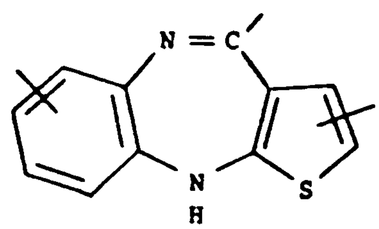

The lead compound from this group, flumezapine, (7-fluoro-2-methyl-10-(4-methyl-1-piperazinyl)-4H-thieno-(2,3-b][1,5]-benzodiazepine), was developed to the stage of being clinically administered to psychiatric patients suffering from schizophrenia. A total of 17 patients received treatment with flumezapine before the clinical trial was terminated after consultation with- the U.S. Food and Drug Administration, because of an unacceptably high incidence of raised enzyme levels in the treated patients. The enzyme, creatinine phosphokinase (CPK), and the liver enzymes, serum glutamate oxalacetic transaminase (SGOT) and serum glutamate pyruvate transaminase (SGPT), estimated from blood samples taken from patients, were in substantial excess of normal values, indicating the possibility of toxicity. In respect of its tendency to raise liver enzyme levels, flumezapine is similar to chlorpromazine, an antipsychotic which has long been in use but whose safety has been called into question.

In clinical trials with flumezapine two of the patients showed the emergence of extra pyramidal side effects as measured on the AIMS scale referred to above.

We have now discovered a compound which possesses surprising and unexpected properties by comparison with flumezapine and other related compounds.

The compound of the invention is of the formula

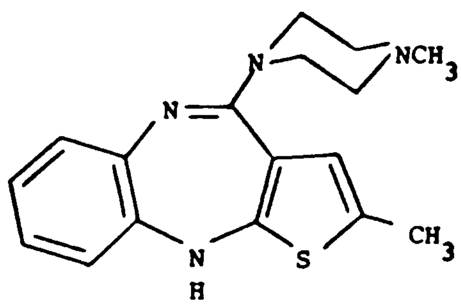

(I)

or an acid addition salt thereof. The free base of formula (I) is 2-methyl-10-(4-methyl-1-piperazinyl)-4H-thieno[2,3-b][1,5]benzodiazepine.

According to an alternative system of nomenclature in which the sulphur atom is designated '1' and numbering is in an anti-clockwise direction in the structure drawn above, the compound of the invention is named 2-methyl-4-(4-methyl-1-piperazinyl)-10H-thieno[2,3-b][1,5]benzodiazepine.

The compound of the invention has given surprising and excellent results, described in greater detail below, in experimental screens for testing activity on the central nervous system and in clinical trials, which results indicate its usefulness for the relatively safe and effective treatment of a wide range of disorders of the central nervous system.

The results of pharmacological tests show that the compound of the invention is an antagonist of dopamine at D-1 and D-2 receptors, and in addition has antimuscarinic anti-cholinergic properties and antagonist activity at 5HT-2 receptor sites. It also has antagonist activity at noradrenergic $\alpha$-receptors. These properties indicate that the compound is a potential neuroleptic with relaxant, anxiolytic or anti-emetic properties, and is useful in treating psychotic conditions such as schizophrenia, schizophreniform diseases and acute mania. At lower doses the compound is indicated for use in the treatment of mild anxiety states.

As mentioned above, the compound of the invention has shown a high level of activity in the clinical evaluation of psychiatric patients suffering from schizophrenia, and it exhibits this high activity at surprisingly low dosage levels. The dosage levels have been found to be lower than would be expected from observations of the compound made in initial tests on animal models. Its response profile in patients follows that of known antipsychotic agents when they have been used successfully, there being a clear similarity between the performance of the compound and that of known antipsychotic agents in its ratings on the major assessment scales such as Brief Psychiatric Rating Scale (BPRS) (Schizophrenia Sub-scale), and Clinical Global Impression (CGI).

In the first completed open (as opposed to blind) study of the compound of the invention in schizophrenic patients, six out of eight patients who completed at least 2 weeks of treatment showed between 66% and 87% improvement at 4 weeks, as assessed on BPRS scale, at daily dosages between 5 and 30 mg. Preliminary results from a further three ongoing clinical trials now appear to confirm this high level of efficacy and at doses lower than or at the low end of the dosage level used in the first study, for example, at 2.5 and 5 mg per day.

Moreover, there is a low incidence of only mild and transient elevation of liver enzymes in patients treated with therapeutic doses, and plasma levels of creatinine phosphokinase (CPK) are lower than with flumezapine, indicating a lower adverse effect on muscular tissue. Furthermore, the compound of the invention causes lower elevation of prolactin levels than other currently used neuroleptic drugs and this suggests fewer disturbances of the menstrual cycle, and less gynecomastia and galactorrhea. No alteration of white blood cell count has been observed in clinical studies.

In dog toxicity studies with a closely analogous compound, 2-ethyl-10-(4-methyl-1-piperazinyl)-4H-thieno[2,3-b]-[1,5]benzodiazepine, at a dosage of 8 mg/kg, it was observed that four out of eight dogs showed a significant rise in cholesterol levels, whereas the compound of the invention did not show any rise in cholesterol levels.

Overall, therefore, in clinical situations, the compound of the invention shows marked superiority, and a better side effects profile than prior known antipsychotic agents, and has a highly advantageous activity level.

The compound of the invention can be used both in its free base and acid addition salt forms. The acid addition salts are preferably the pharmaceutically acceptable, non-toxic addition salts with suitable acids,

such as those of inorganic acids, for example hydrochloric, hydrobromic, nitric, sulphuric or phosphoric acids, or of organic acids, such as organic carboxylic acids, for example glycollic, maleic, hydroxymaleic, fumaric, malic, tartaric, citric or lactic acid, or organic sulphonic acids for example methane sulphonic, ethane sulphonic, 2-hydroxyethane sulphonic, toluene-p-sulphonic or naphthalene-2-sulphonic acid. In addition to pharmaceutically acceptable acid addition salts, other acid addition salts are included in the invention, for example, those with picric or oxalic acid, since they have potential to serve as intermediates in purification or in the preparation of other, for example, pharmaceutically acceptable, acid addition salts, or are useful for identification, characterisation or purification of the free base.

According to a further aspect of the invention there is provided a process for producing a compound of formula (I) or an acid addition salt thereof, which comprises

(a) reacting N-methylpiperazine with a compound of the formula

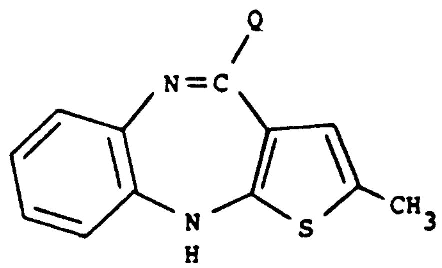

(II)

in which Q is a radical capable of being split off, or

(b) ring-closing a compound of the formula

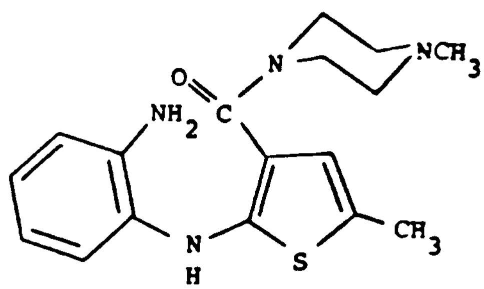

(III)

Appropriate reaction conditions and suitable values of Q can readily be chosen for these processes.

In reaction (a)the radical Q can, for example, be an amino group or a mono- or dialkyl-substituted amino group (each alkyl substituent suitably containing 1 to 4 carbon atoms), hydroxyl, thiol, or an alkoxy, alkylthio or alkylsulphonyl group suitably containing 1 to 4 carbon atoms, for example a methoxy or methylthio group, or a halogen atom, especially a chlorine atom. Preferably, Q is amino ($-NH_2$), hydroxyl or thiol, and amino is most preferred. The reaction is preferably carried out at a temperature of from 50 ° C to 200 ° C.

When Q is amino, the intermediate of formula (II) may also exist in the imino form:

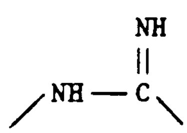

and when Q is hydroxyl or thiol, the intermediates of formula (II) may exist in their amide and thioamide forms:

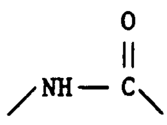

or

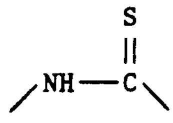

The amidine of formula (II) (Q is $-NH_2$), can be in salt form, for example a salt of a mineral acid such as the hydrochloride, and can be reacted with N-methylpiperazine in an organic solvent such as anisole, toluene, dimethylformamide or dimethyl-sulphoxide, preferably at a temperature range of 100 to 150 °C.

The amidine is prepared by condensing a thiophene compound of formula

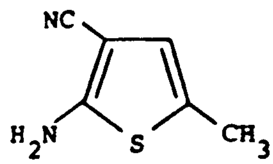

with an ortho-halonitrobenzene, in the presence of a base, for example sodium hydride, in a solvent such as tetrahydrofuran or n-butyl lithium in tetrahydrofuran, or potassium carbonate or lithium hydroxide in dimethylsulphoxide or with a tetraalkylammonium salt in a two-phase system, to form a nitronitrile of formula:

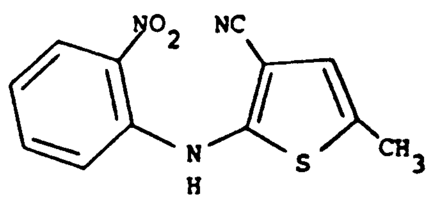

which can be simultaneously reduced and ring-closed to the amidine of formula (II) employing, for example, stannous chloride and hydrogen chloride in aqueous ethanol or, alternatively by reduction with hydrogen and palladium/carbon or ammonium polysulphide followed by acid-catalysed ring closure.

When Q is hydroxyl, reaction (a) is preferably carried out in the presence of titanium tetrachloride which has the ability to react with the N-methylpiperazine to form a metal amine complex. Other metal chlorides such as those of zirconium, hafnium or vanadium may also be employed. The reaction can be carried out in the presence of an acid binding agent such as a tertiary amine, for example, triethylamine.

Alternatively, the reaction can be carried out using excess of N-methylpiperazine to act as an acid-binding agent. A suitable organic solvent such as toluene or chlorobenzene can be used as a reaction medium, although the use of anisole is particularly desirable, at least as a co-solvent, in view of its ability to form a soluble complex with $TiCl_4$.

If desired, elevated temperatures, for example up to 200 °C, can be used to hasten the reaction and a preferred temperature range for carrying out the reaction is from 80 °C to 120 °C.

The intermediate amide of formula (II) (Q is -OH) can be prepared from the corresponding amidine (Q is $-NH_2$) by alkaline hydrolysis, or can be derived from compounds of formula

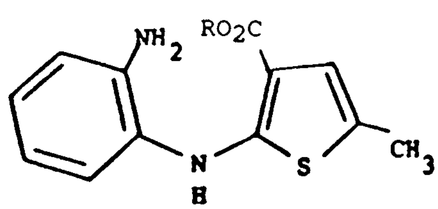

(IV)

in which R is an ester group, preferably $C_{1-4}$ alkyl, by ring closure employing, for example, sodium methylsulphinyl methanide in a suitable solvent such as dimethylsulphoxide. Alternatively, the amide can be prepared by ring closure of an amino-acid, employing for example dicyclo-hexylcarbodiimide (DCC) in a suitable solvent such as tetrahydrofuran. The amino-acid can be obtained for example from the above esters by basic hydrolysis using for example sodium hydroxide in ethanol.

Thioamides of formula (II) (Q is -SH), iminothioethers, iminoethers or iminohalides, or other derivatives containing active Q radicals as specified above, tend to be more reactive towards N-methylpiperazine and can usually be reacted without the necessity for the presence of $TiCl_4$, but otherwise employing the same conditions of temperature and solvent.

The thioamide of formula (II) (Q is -SH) can be prepared by treating a solution of the corresponding amide in an anhydrous basic solvent, such as pyridine, with phosphorous pentasulphide. Similarly, the amide can be converted to the iminothioether, iminoether or iminohalide, or other derivatives containing active Q radicals, by treatment with conventional reagents such as for example in the case of the iminochloride, phosphorous pentachloride.

The intermediate compounds of formula (II) in which Q is a radical capable of being split off, particularly those in which Q is $-NH_2$, -OH or -SH and when Q is $-NH_2$ salts thereof, are novel compounds, and form a further aspect of the present invention.

With regard to reaction (b) above, the compound of formula (III) may be ring-closed by employing, for example, titanium tetrachloride as catalyst and anisole as solvent, and the reaction is preferably carried out at a temperature of 100 °C to 250 °C, for example from 150 °C to 200 °C.

The intermediate compound of formula (III) is preferably prepared <u>in situ</u> without isolation by reacting a compound of formula

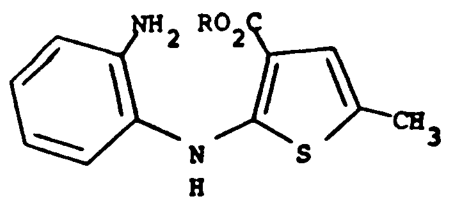

(IV)

in which R is an ester group, preferably $C_{1-4}$ alkyl, with N-methylpiperazine, by heating to a temperature of between 30 °C and 120 °C, for example about 100 °C, in a suitable solvent such as for example anisole, and employing $TiCl_4$ as catalyst.

The compound of formula (IV) can be prepared from the corresponding nitro compound of formula

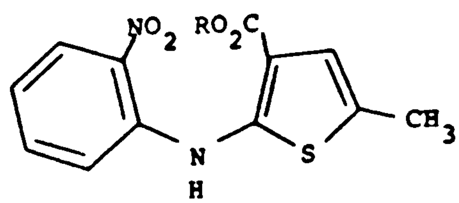

(V)

Such compounds of formula (V) in which R is an ester group, such as for example $C_{1-4}$ alkyl, are novel and form a further aspect of the invention.

If convenient this nitro compound can be converted to the amine of formula (IV) without isolation, before reaction with N-methylpiperazine. Intermediate compounds of formula (V) can be made by condensation of a thiophene of formula

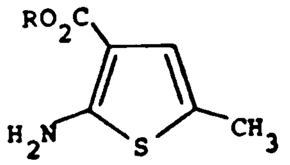

(VI)

with an ortho-halonitrobenzene, preferably ortho fluoro- or chloro- nitrobenzene, in the presence of a base, for example, (a) sodium hydride in a solvent such as for example tetrahydrofuran and at a temperature of from -20°C to 30°C, or (b) anhydrous potassium carbonate or lithium hydroxide in a solvent such as dimethylsulphoxide at a temperature of from 90°C to 120°C. The compound of formula (V) is converted to that of formula (IV) by reduction, for example catalytically, employing hydrogen and palladium/carbon, or chemically, employing stannous chloride and hydrogen chloride in aqueous ethanol, or ammonium polysulphide, or zinc in aqueous ammonium chloride.

It will be appreciated that the compound of formula (I) may be isolated per se or may be converted to an acid addition salt using conventional methods.

As mentioned above, the compound of the invention has useful central nervous system activity. This activity has been demonstrated in models using well-established procedures. For example, the compound has been assessed in a number of standard behavioural tests predictive of antipsychotic activity. It antagonised apomorphine-induced climbing behaviour and hypothermia in mice (Moore, N.A. et al. Psychopharmacology 94 (2), 263-266 (1988), and 96, 539 (1988)) at doses of less than 10 mg/kg. The compound also inhibited a conditioned avoidance response in rats ($ED_{50}$ 4.7 mg/kg), but unlike standard compounds, it induced catalepsy only at much higher doses ($ED_{50}$ 39.4 mg/kg). This separation between the doses required to block a conditioned avoidance response and to induce catalepsy indicates that the compound is less likely to induce extrapyramidal side effects in the clinic

The compound of the invention was also active at doses of less than 10 mg/kg in a test based on the apomorphine-induced climbing test referred to above, which measured the ability of the compound to prevent the disruption of climbing response produced by 24 hour pre-treatment with N-ethoxycarbonyl-2-ethoxy-1,2-dihydroquinoline (EEDQ), a dopamine receptor inactivating agent (Meller et al. Central D1 dopamine receptors, Plenum Press, 1988). This test shows that the compound possesses activity at both the D-1 and D-2 receptors.

In addition, the compound of the invention has been found to have a favourable profile of activity in a number of in vitro binding assays, designed to measure the degree of binding to neural receptors.

In keeping with the observations made in the behavioural tests, the compound is active at both the dopamine D-1 and D-2 receptors as indicated by an $IC_{50}$ of less than 1 $\mu$M in the $^3$H-SCH23390 (Billard, W. et al. Life Sciences 35 1885 (1984)) and the $^3$H-spiperone (Seeman, P. et al. Nature 261 717 (1976)) binding assays respectively.

The compound has an $IC_{50}$ of less than 1 $\mu$M in the $^3$H-QNB binding assay described by Yamamura, HI and Snyder, SH in Proc.Nat.Acad.Sci. USA 71 1725 (1974) indicating that it has antimuscarinic-anticholinergic activity. In addition, the compound shows its greatest activity at the 5-HT-2 receptor in that it displaces H-spiperone from binding sites in the rat frontal cortex (Peroutka, SJ and Snyder, SH Mol. Pharmacol. 16 687 (1979)) at low nanomolar concentrations. The compound is also active at the 5-HT-IC receptor.

This profile of activity in in vitro receptor binding assays, like that observed in the behavioural tests, would indicate that the compound is effective in the treatment of psychotic conditions but is less likely to induce extra pyramidal side-effects.

The compound of the invention is effective over a wide dosage range, the actual dose administered being dependent on the condition being treated. For example, in the treatment of adult humans, dosages of from 0.05 to 30 mg, preferably from 0.1 to 20 mg, per day may be used. A once a day dosage is normally sufficient, although divided doses may be administered. For treatment of psychotic disorders a dose range of from 2 to 15 mg, preferably 2.5 to 10 mg per day is suitable, whereas for mild anxiety states a lower dosage range, such as from 0.1 to 5 mg, preferably 0.5 to 1 mg, may be more appropriate. In choosing a suitable regimen for patients suffering from psychotic illness it may frequently be necessary to begin with a dosage of from 2 to 15 mg per day and when the illness is under control to reduce to a dosage as low as from 0.5 to 1 mg per day. In studies using radiolabelled compound of the invention, residues have been detected in the saliva and thus the compound can potentially be monitored in patients to assess compliance.

The compound of the invention will normally be administered orally or by injection and, for this purpose, it is usually employed in the form of a pharmaceutical composition.

Accordingly the invention includes a pharmaceutical composition comprising as active ingredient a compound of formula (I) or a pharmaceutically acceptable acid addition salt thereof, associated with a pharmaceutically acceptable carrier. In making the compositions of the invention conventional techniques for the preparation of pharmaceutical compositions may be used. For example, the active ingredient will usually be mixed with a carrier, or diluted by a carrier, or enclosed within a carrier which may be in the form of a capsule, sachet, paper or other container. When the carrier serves as a diluent, it may be solid, semi-solid or liquid material which acts as a vehicle, excipient or medium for the active ingredient. The active ingredient can be adsorbed on a granular solid container for example in a sachet. Some examples of suitable carriers are lactose, dextrose, sucrose, sorbitol, mannitol, starches, gum acacia, calcium phosphate, alginates, tragacanth, gelatin, syrup, methyl cellulose, methyl- and propyl-hydroxy-benzoate, talc, magnesium stearate or mineral oil. The compositions of the invention may, if desired, be formulated so as to provide quick, sustained or delayed release of the active ingredient after administration to the patient.

Depending on the method of administration, the compositions may be formulated as tablets, capsules, injection solutions for parenteral use, suspensions or elixirs for oral use or suppositories. Preferably the compositions are formulated in a dosage unit form, each dosage containing from 0.1 to 20 mg, more usually 0.5 to 10 mg, of the active ingredient.

A preferred formulation of the invention is a capsule or tablet comprising 0.1 to 20 mg or 0.5 to 10 mg of active ingredient together with a pharmaceutically acceptable carrier therefor, or a formulation in capsule or tablet form comprising from 2.5 to 5 mg of a compound of formula (I) together with a pharmaceutically acceptable diluent or carrier therefor. A further preferred formulation is an injection which in unit dosage form comprises 0.1 to 20 mg or 0.5 to 10 mg of active ingredient together with a pharmaceutically acceptable diluent therefor. A type of injection formulation that is especially desirable is a sustained release formulation for intra-muscular injection.

The invention is illustrated by the following Examples.

EXAMPLE 1

1. 2-Amino-5-methylthiophene-3-carbonitrile

A mixture of sulphur (217.8 g, 6.79 mol), propionaldehyde (472.5 g, 587 mL, 8.13 mol) and dimethylformamide (1350 m) was placed in a 5 litre flange-necked flask fitted with air stirrer, air condenser, long reach thermometer and dropping funnel. Triethylamine (576 mL, 4.13 mol) was added dropwise over 30 minutes to the cooled stirred reaction mixture whilst maintaining the pot temperature between 5-10 °C with an icebath. After addition was complete the pot was allowed to warm up to 18 °C over 50 minutes, keeping the mixture well stirred. Then a solution of malononitrile (450 g, 6.8 mol) in dimethylformamide (900 mL) was added dropwise over 70 minutes keeping the pot temperature around 20 °C throughout the addition. After addition was complete the mixture was stirred at 15-20 °C for a further 45 minutes then sampled for TLC. The mixture was then poured onto ice (4 litres)/water (8 litres) with stirring and this caused the required product to precipitate. After 10 minutes the stirrer was switched off and the solid allowed to settle. The aqueous liquor was decanted away and the solid isolated by filtration. The isolated solid was well washed with water (de-ionised, 4 litres), then dried over night in vacuo at 70-75 °C to give the title compound (585 g), m.p. 100 °C.

2. 2-(2-Nitroanilino)-5-methylthiophene-3-carbonitrile

To a stirred slurry of sodium hydride (14.4 g, 50% dispersion in oil, 0.3 mol) in dry tetrahydrofuran (50 mL) under nitrogen was added, dropwise, a solution of 2-fluoro-nitrobenzene (28.2 g, 0.2 mol) and 2-amino-5-methylthiophene-3-carbonitrile (27.6 g, 0.2 mol) in dry tetrahydrofuran (250 mL). The mixture was stirred at 25 °C for 24 hours, poured onto cracked ice and extracted into dichloromethane (3 x 500 mL). The combined extracts were washed with 2N hydrochloric acid (2 x 200 mL), water (2 x 200 mL), dried over magnesium sulphate and the solvent removed under reduced pressure. The residue was crystallised from ethanol to give the title compound, (35.2 g), m.p. 99-102 °C.

3. 4-Amino-2-methyl-10H-thieno[2,3-b][1,5]benzodiazepine, hydrochloride

To a stirred slurry of 2-(2-nitroanilino)-5-methyl-thiophene-3-carbonitrile (3 g, 0.011 mol) in ethanol (35 mL) at 50 °C was added, over 10 minutes, a solution of anhydrous stannous chloride (6.95 g, 0.037 mol) in hydrochloric acid (26 mL, 5M). The mixture was stirred under reflux for 1 hour, concentrated under reduced pressure and allowed to crystallise over night at 5 °C. The salt was filtered, washed with a small amount of water, dried (4.3 g) m.p. >250 °C, and used without further purification in the next stage.

4. 2-Methyl-10-(4-methyl-1-piperazinyl)-4H-thieno[2,3-b][1,5]-benzodiazepine

Crude 4-amino-2-methyl-10H-thieno[2,3-b][1,5]benzodiazepine, hydrochloride (4.3 g) was refluxed in a mixture of N-methylpiperazine (15 mL), dimethylsulphoxide (20 mL) and toluene (20 mL) under a nitrogen atmosphere for 20 hours. The mixture was cooled to ca. 50 °C, water (20 mL) added, and the product allowed to crystallise at 5 °C over night. The product was filtered and crystallised from acetonitrile (30 mL) to give the title compound (1.65g) m.p. 195 °C.

The structure of the compound was confirmed spectroscopically:

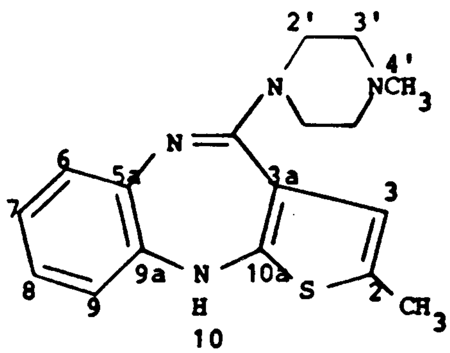

$^1$H NMR ($CDCl_3$): δ 2.30 (3H, s, 4'-$CH_3$), 2.28 (3H, s, 2-$CH_3$), 2.45 (4H, m, 3'-$CH_2$) 3.49 (4H, m, 2'-$CH_2$), 5.00 (H, broad s, 10-NH), 6.23 (H, broad s, 3-CH), 6-35-7-10 (4H, m, 6,7,8,9-H).

$^3$C NMR ($CDCl_3$): δ 128.5 (s, C-2), 127.8 (d, C-3), 119.1 (s, C-3a), 157.4 (s, C-4) 140.8 (s, C-5a), 123.4, 122.6, 124.1 (d, C-6,7,8), 118.8 (d, C-9), 142.5 (s, C-9a), 151.8 (s, C-10a), 46.5 (t, 2'-C), 54.8 (t, 3'-C) 45.9 (q, -4'-C), 15.2 (q, 2-Me).

Mass spectra shows an M+ of 312 and major fragment ions of m/z 255, 242, 229 and 213.

EXAMPLE 2

1. Methyl 2-amino-5-methylthiophene-3-carboxylate

To a stirred mixture of methyl cyanoacetate (3.9 g, 0.04 mol), sulphur (1.26 g, 0.04 mol) and triethylamine (3.2 mL, 002 mol) in dry methylformamide (12 mL) under a nitrogen atmosphere at 45 °C was added, dropwise, a solution of freshly distilled propionaldehyde (2.5 g, 0.043 mol) in dry dimethylformamide (2 mL), keeping the temperature at 45-47 °C. The mixture was stirred at 45 °C for 1.5 hours, then partitioned between water and ethyl acetate. The organic extract was washed with water, dried and evaporated. The title compound was purified by chromatography on neutral alumina, eluting with chloroform-hexane (4.8 g).

2. Methyl 2-(2-nitroanilino)-5-methylthiophene-3-carboxylate

To a stirred suspension of sodium hydride (2 g) in dry tetrahydrofuran (25 mL) under a nitrogen atmosphere was added a solution of methyl 2-amino-5-methylthiophene-3-carboxylate (4.8 g, 0.028 mol) and 2-fluoronitrobenzene (4.0 g, 0.025 mol) in dry tetrahydrofuran (30 mL). The mixture was stirred at 25 °C for 20 hours, poured onto ice and partitioned between 2N hydrochloric acid and ethyl acetate. The organic extracts were dried over magnesium sulphate, the solvent was evaporated under reduced pressure and the title compound purified by chromatography on silica gel, eluted with toluene, and crystallised from ethanol (4.1 g), m.p. 123-125 °C.

3. 2-Methyl-10-(4-methyl-1-piperazinyl)-4H-thieno[2,3-b][1,5]-benzodiazepine

Methyl 2-(2-nitroanilino)-5-methylthiophene-3-carboxylate (3.7 g, 0.0013 mol) was hydrogenated in a Parr apparatus at 60 psi in ethanol-ethyl acetate (2:1, 150 mL) with palladium on charcoal catalyst (10%, 200 mg). After removal of catalyst and solvent the crude diamino-ester was dissolved in a mixture of N-methylpiperazine (21 mL) and anisole (55 mL). To this solution, under a nitrogen atmosphere was added, with stirring, a solution of titanium tetrachloride (3.45 mL) in anisole (15 mL). The mixture was stirred at 100°C for 1 hour, then under reflux for 48 hours to effect ring closure of 1-{[2-(2-amino-anilino)-5-methylthiophen-3-yl]carbonyl}-4-methylpiperazine.

After allowing to cool to 80°C a mixture of 30% ammonia solution (10 mL) and isopropanol (10 mL) was cautiously added, followed by ethyl acetate (25 mL). The inorganic precipitate was removed by filtration and the filtrate washed with water (3 x 25 mL), dried with magnesium sulphate and the solvent removed under reduced pressure. The product was purified by chromatography on Florisil, eluted with ethyl acetate and finally crystallised from acetonitrile (40 mL) to give the title compound (2.32 g), identical with that described above.

EXAMPLE 3

A pulvule formulation is prepared by blending the active with silicone starch, and filling it into hard gelatin capsules.

| | Per 300 mg capsule |
|---|---|
| Compound of the invention | 5.0 mg |
| Silicone | 2.9 mg |
| Starch flowable | 292.1 mg |

EXAMPLE 4

A tablet formulation is made by granulating the active with appropriate diluent, lubricant, disintegrant and binder and compressing

| | |
|---|---|
| Compound of the invention | 5.0 mg |
| Magnesium stearate | 0.9 mg |
| Microcrystalline cellulose | 75.0 mg |
| Povidone | 15.0 mg |
| Starch, directly compressible | 204.1 mg |

EXAMPLE 5

An aqueous injection of active is prepared as a freeze-dried plug, for reconstitution in a suitable, sterile diluent before use (to a total volume of 10 ml).

| | |
|---|---|
| Compound of the invention<br>Mannitol | 20.0 mg<br>20.0 mg |
| N Hydrochloric acid and/or N sodium hydroxide to adjust pH to 5-5.5. | |

EXAMPLE 6

A controlled release injection for intramuscular injection is formed from a sterile suspension of micronised active in an oleaginous vehicle.

| | |
|---|---|
| Compound of the invention | 65.0 mg |
| Aluminium stearate | 0.04 mg |
| Sesame oil | 2 ml |

EXAMPLE 7

A formulation is prepared by blending the active with silicone starch and starch, and filling it into hard gelatine capsules.

| | Per 290 mg capsule |
|---|---|
| Compound of the invention | 2.5 mg |
| Starch flowable with 0.96% silicone 220 | 217.5 mg |
| Starch flowable | 70.0 mg |

## Claims

**Claims for the following Contracting States : AT, BE, CH, DE, DK, FR, GB, IT, LI, LU, NL, SE**

**1.** 2-Methyl-10-(4-methyl-1-piperazinyl)-4H-thieno[2,3-b][1,5]benzodiazepine, or an acid addition salt thereof.

**2.** 2-Methyl-10-(4-methyl-1-piperazinyl)-4H-thieno[2,3-b][1,5]benzodiazepine, or a pharmaceutically acceptable acid addition salt thereof.

**3.** 2-Methyl-10-(4-methyl-1-piperazinyl)-4H-thieno-[2,3-b][1,5]benzodiazepine.

**4.** A compound according to claim 2 or 3 for use as a pharmaceutical.

**5.** The use of a compound according to claim 2 or 3 for the manufacture of a medicament for the treatment of a disorder of the central nervous system.

**6.** The use of a compound according to claim 2 or 3 for the manufacture of a medicament for the treatment of schizophrenia.

**7.** The use of a compound according to claim 2 or 3 for the manufacture of a medicament for the treatment of a schizophreniform disease.

**8.** The use of a compound according to claim 2 or 3 for the manufacture of a medicament for the treatment of acute mania.

**9.** The use of a compound according to claim 2 or 3 for the manufacture of a medicament for the treatment of mild anxiety states.

10. A pharmaceutical composition comprising a compound according to claim 2 together with a pharmaceutically acceptable diluent or carrier therefor.

11. A dosage unit form comprising from 0.1 to 20 mg of a compound according to claim 2.

12. A dosage unit form comprising from 0.5 to 10 mg of a compound according to claim 2.

13. A pharmaceutical composition comprising the compound of claim 3 together with a pharmaceutically acceptable diluent or carrier therefor.

14. A pharmaceutical composition in capsule or tablet form comprising 0.1 to 20 mg of the compound of claim 3.

15. A pharmaceutical composition in capsule or tablet form comprising 0.5 to 10 mg of the compound of claim 3.

16. A pharmaceutical composition in capsule or tablet form comprising from 2.5 to 5 mg of the compound of claim 3 together with a pharmaceutically acceptable diluent or carrier therefor.

17. A pharmaceutical injection which in unit dosage form comprises 0.1 to 20 mg of a compound according to claim 2 or 3.

18. A pharmaceutical injection which in unit dosage form comprises 0.5 to 10 mg of a compound according to claim 2 or 3.

19. A pharmaceutical injection according to claim 17 or 18 which is a sustained release formulation for intra-muscular injection.

20. A process for producing a compound according to claim 1, which comprises
(a) reacting N-methylpiperazine with a compound of the formula

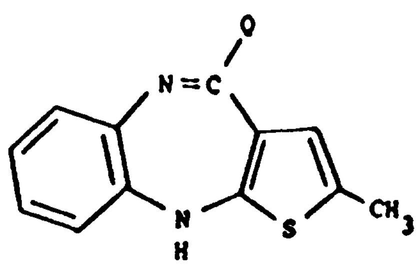

in which Q is a radical capable of being split off, or
(b) ring-closing a compound of the formula

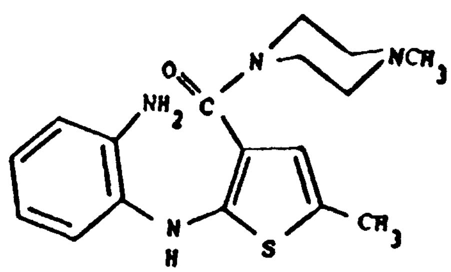

**21.** A compound of the formula

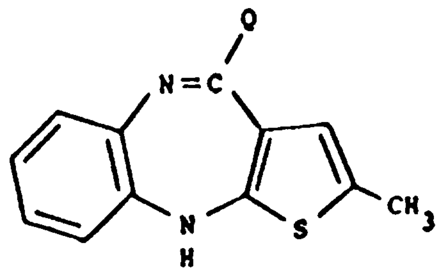

in which Q is $-NH_2$, -OH or -SH, and when Q is $-NH_2$ salts thereof.

**22.** A compound according to claim 21 in which Q is $-NH_2$, or a salt thereof.

**Claims for the following Contracting State : ES**

**1.** A process for producing 2-methyl-10-(4-methyl-1-piperazinyl)-4H-thieno[2,3-b][1,5]benzodiazepine, or an acid salt thereof, which comprises

(a) reacting N-methylpiperazine with a compound of the formula

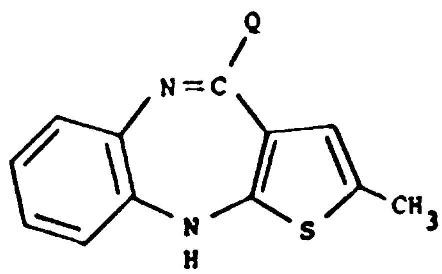

in which Q is a radical capable of being split off, or

(b) ring-closing a compound of the formula

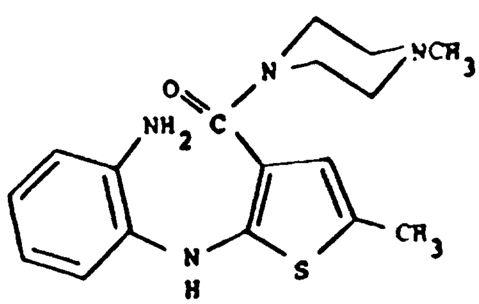

**2.** A process according to claim 1 for producing 2-methyl-10-(4-methyl-1-piperazinyl)-4H-thieno[2,3-b]-[1,5] benzodiazepine, or an acid salt thereof, which comprises reacting N-methylpiperazine with a compound of the formula

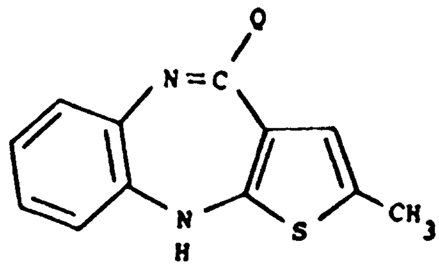

in which Q is $-NH_2$, -OH or -SH, and when Q is $NH_2$ salts thereof.

3. A process according to claim 2 which comprises reacting a compound in which Q is $NH_2$ or a salt thereof.

4. A process according to either of claims 2 and 3, which is carried out at a temperature of from 50° C. to 200° C.

5. A process for the preparation of a pharmaceutical composition which comprises admixing 2-methyl-10-(4-methyl-1-piperazinyl)-4H-thieno[2,3-b][1,5]benzodiazepine, or an acid salt thereof, together with a pharmaceutically acceptable diluent or carrier therefor.

**Claims for the following Contracting State : GR**

1. A process for producing 2-methyl-10-(4-methyl-1-piperazinyl)-4H-thieno[2,3-b][1,5]benzodiazepine, or an acid salt thereof, which comprises

(a) reacting N-methylpiperazine with a compound of the formula

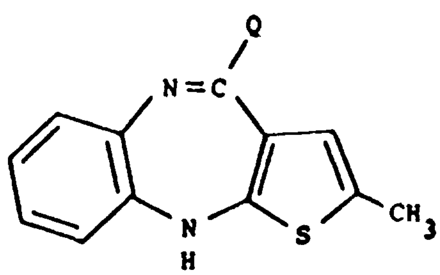

in which Q is a radical capable of being split off, or

(b) ring-closing a compound of the formula

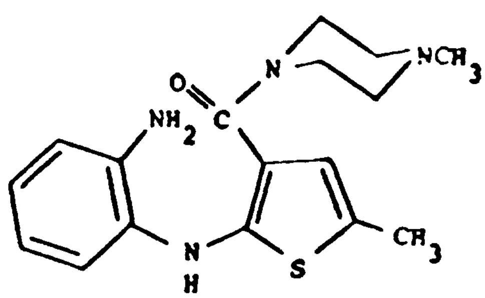

2. A process for producing 2-methyl-10-(4-methyl-1-piperazinyl)-4H-thieno[2,3-b][1,5]benzodiazepine, or an acid salt thereof, which comprises reacting N-methylpiperazine with a compound of the formula

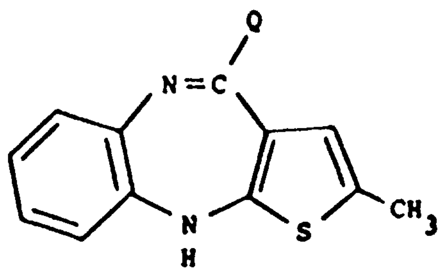

in which Q is $-NH_2$, -OH or -SH, and when Q is $NH_2$ salts thereof.

3. A process according to claim 2 which comprises reacting a compound in which Q is $NH_2$ or a salt thereof.

4. A process according to either of claim 2 and 3, which is carried out at a temperature of from 50° C. to 200° C.

5. A compound of the formula

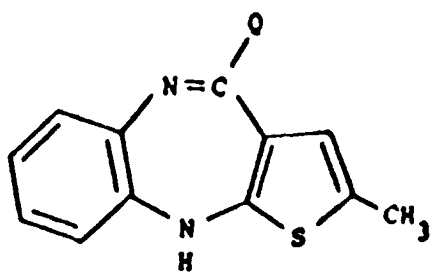

in which Q is $-NH_2$, -OH or -SH, and when Q is $-NH_2$ salts thereof.

6. A compound according to claim 5 in which Q is $-NH_2$, or a salt thereof.

**Patentansprüche**
**Patentansprüche für folgende Vertragsstaaten : AT, BE, CH, DE, DK, FR, GB, IT, LI, LU, NL, SE**

1. 2-Methyl-10-(4-methyl-1-piperazinyl)-4H-thieno-[2,3-b] [1,5]benzodiazepin oder ein Säureadditionssalz davon.

2. 2-Methyl-10-(4-methyl-1-piperazinyl)-4H-thieno-[2,3-b] [1,5]benzodiazepin oder ein pharmazeutisch brauchbares Säureadditionssalz davon.

3. 2-Methyl-10-(4-methyl-1-piperazinyl)-4H-thieno-[2,3-b] [1,5]benzodiazepin.

4. Verbindung nach Anspruch 2 oder 3 zur Verwendung als Arzneimittel.

5. Verwendung einer Verbindung nach Anspruch 2 oder 3 zur Herstellung eines Arzneimittels zur Behandlung einer Störung im Zentralnervensystem.

6. Verwendung einer Verbindung nach Anspruch 2 oder 3 zur Herstellung eines Arzneimittels zur Behandlung von Schizophrenie.

7. Verwendung einer Verbindung nach Anspruch 2 oder 3 zur Herstellung eines Arzneimittels zur Behandlung einer schizophreniformen Krankheit.

**8.** Verwendung einer Verbindung nach Anspruch 2 oder 2 zur Herstellung eines Arzneimittels zur Behandlung von akuter Manie.

**9.** Verwendung einer Verbindung nach Anspruch 2 oder 3 zur Herstellung eines Arzneimittels zur Behandlung von leichten Angstzuständen.

**10.** Pharmazeutische Zusammensetzung, umfassend eine Verbindung nach Anspruch 2, zusammen mit einem pharmazeutisch brauchbaren Verdünnungsmittel oder Träger dafür.

**11.** Dosiseinheitsform, umfassend 0.1 bis 20 mg einer Verbindung nach Anspruch 2.

**12.** Dosiseinheitsform, umfassend 0.5 bis 10 mg einer Verbindung nach Anspruch 2.

**13.** Pharmazeutische Zusammensetzung, umfassend die Verbindung nach Anspruch 3 zusammen mit einem pharmazeutisch brauchbaren Verdünnungsmittel oder Träger dafür.

**14.** Pharmazeutische Zusammensetzung in Kapsel- oder Tablettenform, umfassend 0.1 bis 20 mg der Verbindung von Anspruch 3.

**15.** Pharmazeutische Zusammensetzung in Kapsel- oder Tablettenform, umfassend 0.5 bis 10 mg der Verbindung von Anspruch 3.

**16.** Pharmazeutische Zusammensetzung in Kapsel- oder Tablettenform, umfassend 2.5 bis 5 mg der Verbindung von Anspruch 3 zusammen mit einem pharmazeutisch brauchbaren Verdünnungsmittel oder Träger dafür.

**17.** Pharmazeutische Injektion, die in der Dosiseinheitsform 0.1 bis 20 mg einer Verbindung nach Anspruch 2 oder 3 umfaßt.

**18.** Pharmazeutische Injektion, die in der Dosiseinheitsform 0.5 bis 10 mg einer Verbindung nach Anspruch 2 oder 3 umfaßt.

**19.** Pharmazeutische Injektion nach Anspruch 17 oder 18, die eine langzeitwirkende Formulierung für intramuskuläre Injektionen ist.

**20.** Verfahren zur Herstellung einer Verbindung nach Anspruch 1, umfassend
(a) die Umsetzung von N-Methylpiperazin mit einer Verbindung der Formel,

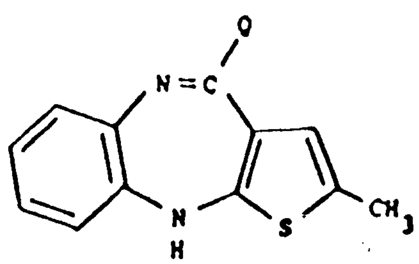

wobei Q ein abspaltbarer Rest ist, oder

(b) den Ringschluß einer Verbindung der Formel

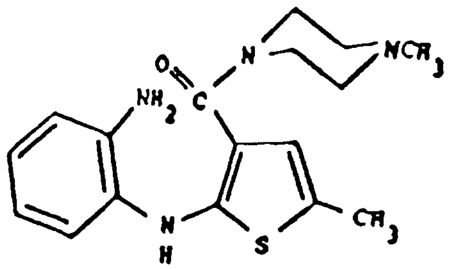

**21.** Verbindung der Formel,

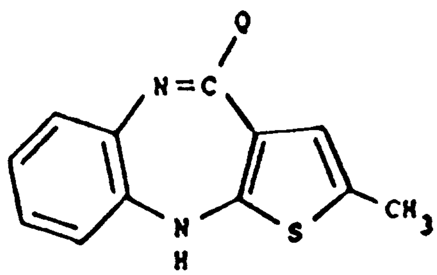

wobei Q $-NH_2$, -OH oder -SH ist und, wenn Q $-NH_2$ ist, Salze davon.

**22.** Verbindung nach Anspruch 21, wobei Q $-NH_2$ ist oder ein Salz davon.

**Patentansprüche für folgenden Vertragsstaat : ES**

**1.** Verfahren zur Herstellung von 2-Methyl-10-(4-methyl-1-piperazinyl)-4H-thieno[2,3-b][1,5]benzodiazepin oder ein Säuresalz davon, umfassend

(a) die Umsetzung von N-Methylpiperazin mit einer Verbindung der Formel,

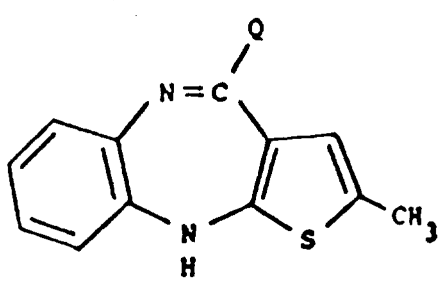

wobei Q ein abspaltbarer Rest ist oder

(b) den Ringschluß einer Verbindung der Formel

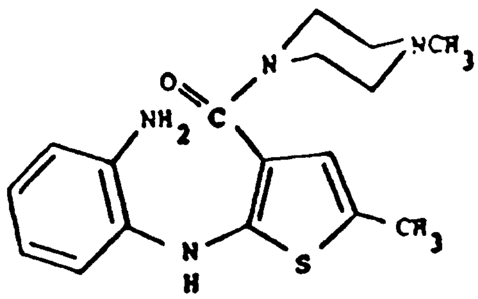

2. Verfahren nach Anspruch 1 zur Herstellung von 2-Methyl-10-(4-methyl-1-piperazinyl)-4H-thieno[2,3-b]-[1,5]benzodiazepin oder ein Säuresalz davon, umfassend die Umsetzung von N-Methylpiperazin mit einer Verbindung der Formel,

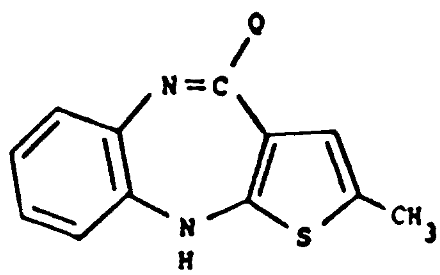

wobei Q $-NH_2$, -OH oder -SH ist und wenn Q $NH_2$ ist, Salze davon.

3. Verfahren nach Anspruch 2, umfassend die Umsetzung einer Verbindung in der Q $NH_2$ ist oder eines Salzes davon.

4. Verfahren nach einem der Ansprüche 2 und 3, das bei einer Temperatur von 50°C bis 200°C durchgeführt wird.

5. Verfahren zur Herstellung einer pharmazeutischen Zusammensetzung, daß das Vermischen von 2-Methyl-10-(4-methyl-1-piperazinyl)-4H-thieno[2,3-b][1,5]benzodiazepin oder ein Säuresalz davon mit einem pharmazeutisch brauchbaren Verdünnungsmittel oder Träger dafür umfaßt.

**Patentansprüche für folgenden Vertragsstaat : GR**

1. Verfahren zur Herstellung von 2-Methyl-10-(4-methyl-1-piperazinyl)-4H-thieno[2,3-b][1,5]benzodiazepin oder ein Säuresalz davon, umfassend

(a) die Umsetzung von N-Methylpiperazin mit einer Verbindung der Formel,

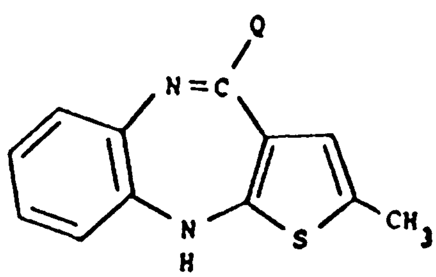

wobei Q ein abspaltbarer Rest ist oder

(b) den Ringschluß einer Verbindung der Formel

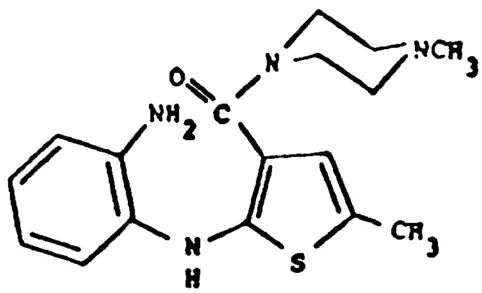

2. Verfahren zur Herstellung von 2-Methyl-10-(4-methyl-1-piperazinyl)-4H-thieno[2,3-b][1,5]benzodiazepin oder ein Säuresalz davon, umfassend die Umsetzung von N-Methylpiperazin mit einer Verbindung der Formel,

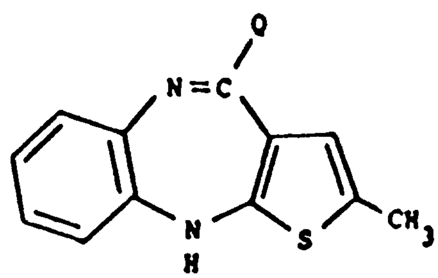

wobei Q $-NH_2$, -OH oder -SH ist und, wenn Q $NH_2$ ist, Salze davon.

3. Verfahren nach Anspruch 2, umfassend die Umsetzung einer Verbindung, worin Q $NH_2$ ist oder eines Salzes davon.

4. Verfahren nach einem der Ansprüche 2 und 3, das bei einer Temperatur von 50°C bis 200°C durchgeführt wird.

5. Verbindung der Formel,

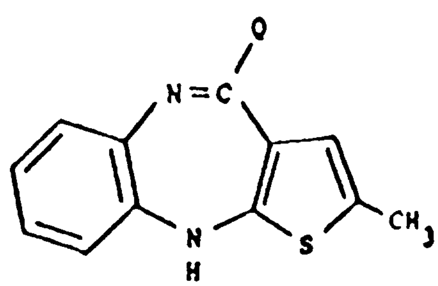

wobei Q $-NH_2$, -OH oder -SH ist und, wenn Q $NH_2$ ist, Salze davon.

6. Verbindung nach Anspruch 5, wobei Q $-NH_2$ ist oder ein Salz davon.

**Revendications**

**Revendications pour les Etats contractants suivants : AT, BE, CH, DE, DK, FR, GB, IT, LI, LU, NL, SE**

1. 2-méthyl-10-(4-méthyl-1-pipérazinyl)-4H-thiéno[2,3-b][1,5]benzodiazépine, ou sel d'addition acide de celle-ci.

2. 2-méthyl-10-(4-méthyl-1-pipérazinyl)-4H-thiéno[2,3-b][1,5]benzodiazépine, ou sel d'addition acide pharmaceutiquement acceptable de celle-ci.

3. 2-méthyl-10-(4-méthyl-1-pipérazinyl)-4H-thiéno[2,3-b][1,5]benzodiazépine.

4. Composé selon la revendication 2 ou 3 pour l'utilisation comme produit pharmaceutique.

5. Utilisation d'un composé selon la revendication 2 ou 3 pour la préparation d'un médicament pour le traitement d'un désordre du système nerveux central.

6. Utilisation d'un composé selon la revendication 2 ou 3 pour la préparation d'un médicament pour le traitement de la schizophrénie.

7. Utilisation d'un composé selon la revendication 2 ou 3 pour la préparation d'un médicament pour le traitement d'une maladie schizophréniforme.

8. Utilisation d'un composé selon la revendication 2 ou 3 pour la préparation d'un médicament pour le traitement de la manie aigue.

9. Utilisation d'un composé selon la revendication 2 ou 3 pour la préparation d'un médicament pour le traitement d'états d'anxiété modérée.

10. Composition pharmaceutique comprenant un composé conforme à la revendication 2, ensemble avec un diluant ou un support pharmaceutiquement acceptable pour celui-ci.

11. Forme posologique unitaire comprenant de 0,1 à 20 mg d'un composé conforme à la revendication 2.

12. Forme posologique unitaire comprenant de 0,5 à 10 mg d'un composé conforme à la revendication 2.

13. Composition pharmaceutique comprenant le composé selon la revendication 3, ensemble avec un diluant ou un support pharmaceutiquement acceptable pour celui-ci.

14. Composition pharmaceutique sous forme de capsule ou de comprimé comprenant 0,1 à 20 mg du composé selon la revendication 3.

15. Composition pharmaceutique sous forme de capsule ou de comprimé comprenant 0,5 à 10 mg du composé selon la revendication 3.

16. Composition pharmaceutique sous forme de capsule ou de comprimé comprenant de 2,5 à 5 mg du composé selon la revendication 3, ensemble avec un diluant ou un support pharmaceutiquement acceptable pour celui-ci.

17. Injection pharmaceutique qui, sous forme posologique unitaire, comprend 0,1 à 20 mg d'un composé selon la revendication 2 ou 3.

18. Injection pharmaceutique qui, sous forme posologique unitaire, comprend 0,5 à 10 mg d'un composé selon la revendication 2 ou 3.

19. Injection pharmaceutique selon la revendication 17 ou 18 qui est une formulation à libération prolongée pour l'injection par voie intramusculaire.

20. Procédé pour la production d'un composé selon la revendication 1, qui comprend

(a) la mise en réaction de la N-méthylpipérazine avec un composé répondant à la formule

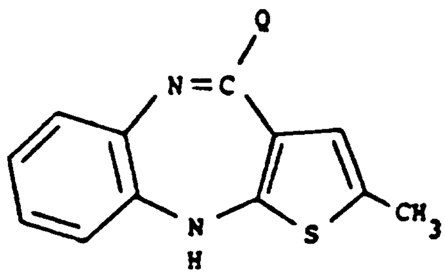

dans laquelle Q est un radical susceptible d'être éliminé, ou
(b) la fermeture du cycle d'un composé répondant à la formule

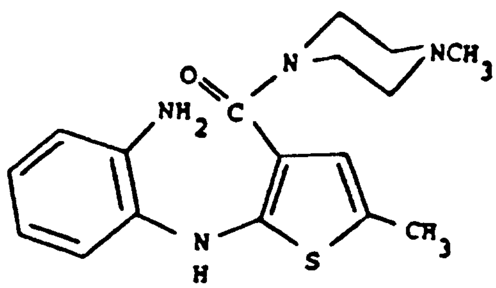

**21.** Composé répondant à la formule

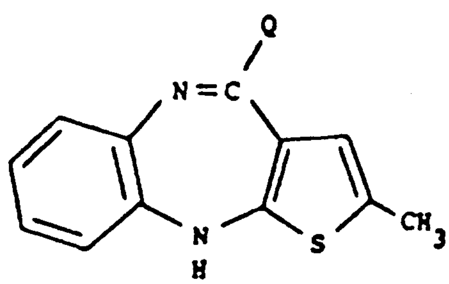

dans laquelle Q représente $-NH_2$, -OH ou -SH, et lorsque Q représente - $NH_2$, les sels de celui-ci.

**22.** Composé selon la revendication 21, dans laquelle Q représente $-NH_2$, ou un sel de celui-ci.

**Revendications pur l'Etat contractant suivant : ES**

**1.** Procédé pour la production de la 2-méthyl-10-(4-méthyl-1-pipérazinyl)-4H-thiéno[2,3-b][1,5]-benzodiazépine, ou d'un sel acide de celle-ci, qui comprend :

(a) la mise en réaction de la N-méthylpipérazine avec un composé répondant à la formule

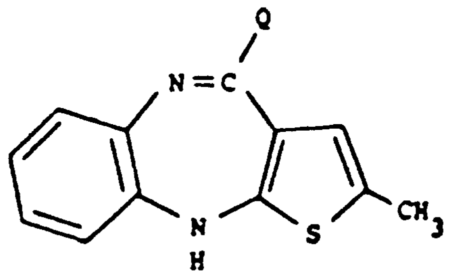

dans laquelle Q est un radical susceptible d'être éliminé, ou

(b) la fermeture du cycle d'un composé répondant à la formule

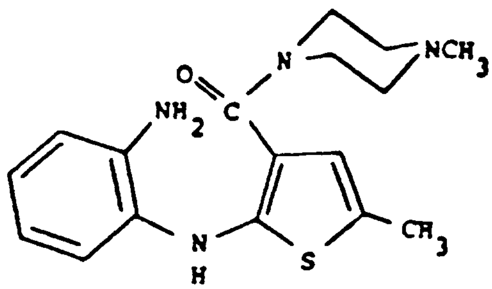

2. Procédé selon la revendication 1 pour la production de la 2-méthyl-10-(4-méthyl-1-pipérazinyl)-4H-thiéno[2,3-b][1,5]benzodiazépine ou d'un sel acide de celle-ci, qui comprend la mise en réaction de la N-méthylpipérazine avec un composé répondant à la formule

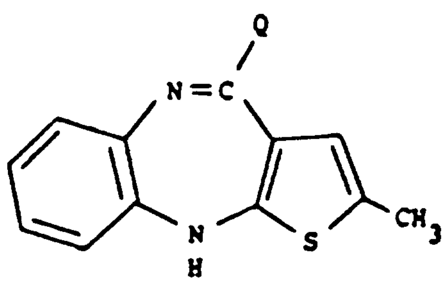

dans laquelle Q représente $-NH_2$, -OH ou -SH, et lorsque Q représente $-NH_2$, les sels de celle-ci.

3. Procédé selon la revendication 2, qui comprend la mise en réaction d'un composé dans lequel Q représente $-NH_2$ ou un sel de celui-ci.

4. Procédé selon l'une ou l'autre des revendications 2 et 3, qui est effectué à une température de 50 °C à 200 °C.

5. Procédé pour la préparation d'une composition pharmaceutique qui comprend le mélange de la 2-méthyl-10-(4-méthyl-1-pipérazinyl)-4H-thiéno[2,3-b][1,5]benzodiazépine, ou d'un sel acide de celle-ci, ensemble avec un diluant ou un support pharmaceutiquement acceptable pour celle-ci.

**Revendications pour l'Etat contractant suivant : GR**

1. Procédé pour la production de la 2-méthyl-10-(4-méthyl-1-pipérazinyl)-4H-thiéno[2,3-b][1,5]-benzodiazépine, ou d'un sel acide de celle-ci, qui comprend :
   (a) la mise en réaction de la N-méthylpipérazine avec un composé répondant à la formule

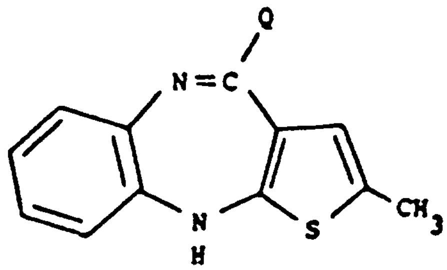

   dans laquelle Q est un radical susceptible d'être éliminé, ou
   (b) la fermeture du cycle d'un composé répondant à la formule

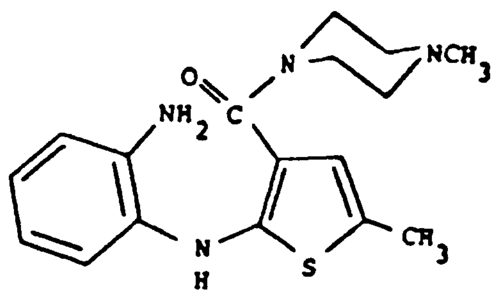

2. Procédé pour la production de la 2-méthyl-10-(4-méthyl-1-pipérazinyl)-4H-thiéno[2,3-b][1,5]-benzodiazépine, ou d'un sel acide de celle-ci, qui comprend la mise en réaction de la N-méthylpipérazine avec un composé répondant à la formule

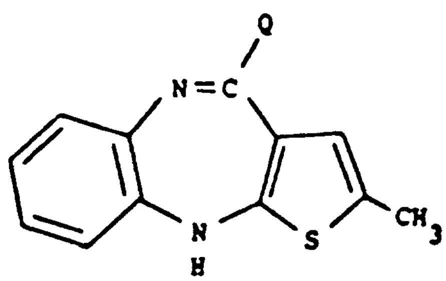

   dans laquelle Q représente $-NH_2$, -OH ou -SH, et lorsque Q représente $-NH_2$, les sels de celle-ci.

3. Procédé selon la revendication 2, qui comprend la mise en réaction d'un composé dans lequel Q représente $-NH_2$ ou un sel de celui-ci.

4. Procédé selon l'une ou l'autre des revendications 2 et 3, qui est effectué à une température de 50 °C à 200 °C.

**5.** Composé répondant à la formule

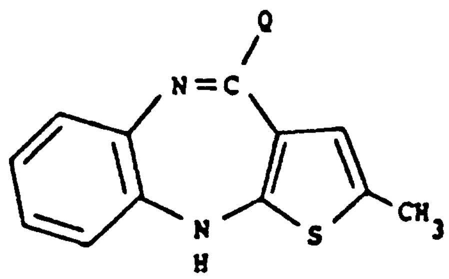

dans laquelle Q représente $-NH_2$, -OH ou -SH, et lorsque Q représente - $NH_2$, les sels de celui-ci.

**6.** Composé selon la revendication 5, dans lequel Q représente $-NH_2$, ou un sel de celui-ci.